# EUROPEAN PATENT SPECIFICATION

(11) **EP 3 943 502 B1**
(45) Date of publication and mention of the grant of the patent: **21.05.2025**
(21) Application number: 20773082.1
(22) Date of filing: 19.03.2020
(51) Int. Cl.: C07J 9/00, C07J 41/00, C07J 51/00

(54) **METHOD FOR PREPARING CHENODEOXYCHOLIC ACID DERIVATIVE**
VERFAHREN ZUR HERSTELLUNG EINES CHENODEOXYCHOLINSÄUREDERIVATS
PROCÉDÉ DE PRÉPARATION D'UN DÉRIVÉ D'ACIDE CHÉNODÉSOXYCHOLIQUE

(30) Priority: 19.03.2019 CN 201910210103
(43) Date of publication of application: 26.01.2022
(73) Proprietor: Suzhou Zelgen Biopharmaceutical Co., Ltd., Jiangsu 215300 (CN)
(72) Inventor: LV, Binhua, Suzhou, Jiangsu 215300 (CN); LI, Chengwei, Suzhou, Jiangsu 215300 (CN); GUO, Chao, Suzhou, Jiangsu 215300 (CN)
(74) Representative: dompatent von Kreisler Selting Werner - Partnerschaft von Patent- und Rechtsanwälten mbB
(86) International application number: PCT/CN2020/080244
(87) International publication number: WO 2020/187298

(56) References cited:
- WO-A1-2019/170521
- WO-A1-2020/039449
- CN-A- 105 669 811
- CN-A- 106 279 328
- CN-A- 106 632 564
- CN-A- 108 456 238
- US-A1- 2018 099 991

## Description

### FIELD OF THE INVENTION

The invention belongs to the pharmaceutical field. Specifically, the present invention relates to a novel preparation and purification method of chenodeoxycholate.

### BACKGROUND OF THE INVENTION

Farnesoid X Receptor (FXR) is a member of the nuclear receptor family. It is mainly expressed in the liver, small intestine and other intestinal systems, and is involved in bile acid metabolism and cholesterol metabolism. Bile acids have a variety of physiological functions and play an important role in processes such as fat absorption, transport, distribution and cholesterol homeostasis. The farnesoid X receptor acts as a receptor for bile acids such as chenodeoxycholic acid, maintains the balance of bile acids in the body by regulating the expression of genes involved in bile acid metabolism. In addition, the farnesoid X receptor also plays an important role in the dynamic balance of glucose in the body and insulin resistance. Therefore, farnesoid X receptor agonists are expected to be developed into drugs for treatment of non-alcoholic steatohepatitis, non-alcoholic fatty liver disease, gallstones, primary biliary cirrhosis, liver cirrhosis, liver fibrosis, diabetes, hypercholesterolemia, atherosclerosis, obesity, hypertriglyceridemia, etc.. Among them, the compound obeticholic acid is a selective farnesoid X receptor agonist, of which the chemical name is 3α,7α-dihydroxy-6α-ethyl-5β-cholan-24-oic acid. It is useful in treating primary biliary cirrhosis (PBC), non-alcoholic steatohepatitis (NASH) and non-alcoholic fatty liver related diseases. At present, obeticholic acid has been approved to market in primary biliary cirrhosis, and it is in phase TTT clinical research for NASH

Patent application WO02072598 discloses a method for synthesizing chenodeoxycholic acid derivatives by using 3α-dihydroxyl-7-keto-5β-cholan-24-oic acid as a starting material through steps such as 6-position alkylation. However, there are many shortcomings in this synthetic method, for example, all intermediates and products need to be purified by chromatographic column, the total yield of the reaction is very low (only 3.5%), and carcinogenic reagents should be used in the reaction step.

In addition, the patent WO2006122977 discloses a method for synthesizing obeticholic acid using 3α-dihydroxyl-7-keto-5β-cholan-24-oic acid as a starting material through Aldol condensation and other steps. However, the synthesis method have many shortcomings such as the intermediates are not easy to separate, long reaction steps, low yield of the configuration conversion step, and the impurities in the final product are not easy to remove.

CN108456238 discloses a method for the preparation of obeticholic acid via an N-heteroaryl-amide intermediate or an amide intermediate, wherein the N atom thereof is part of a heterocyclic group.

Therefore, there is still need in developing better method for synthesizing chenodeoxycholate.

### BRIEF SUMMARY OF THE INVENTION

In the first aspect of the present invention, a method for synthesizing the compound represented by Formula (A) is provided: 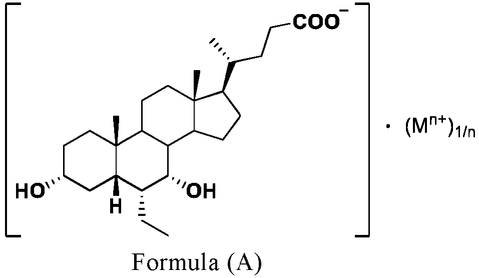
wherein, n is 1 or 2, and M is NH₄⁺, alkali metal ion, alkaline earth metal ion or transition metal ion;
the method comprises the following steps:
   (a) in an inert solvent, subjecting the compound of Formula (VI) to the catalytic hydrogenation and reduceing with metal hydride reducing agent by "one-pot method" 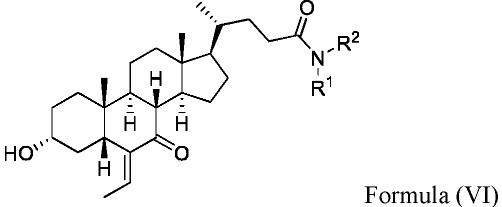 to obtain the compound of Formula (V); 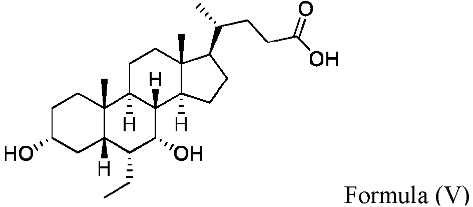
   (b) salifying compound of Formula (V) and crystallizing in a solvent to obtain the compound of Formula (A);
wherein, R¹ and R² are both hydrogen or R¹ is hydrogen and R² is hydroxyl.

In another preferred embodiment, during the process of "one-pot method" of catalytic hydrogenation and metal hydride reducing agent reduction, the intermediate product is not separated or purified.

In another preferred embodiment, the step (a) includes: in anhydrous methanol, the compound of Formula (VI) is used for catalytic hydrogenation, and then water and metal hydride are added to react to obtain a compound of Formula (V).

In another preferred embodiment, the compound represented by Formula (VI) is the following compound:

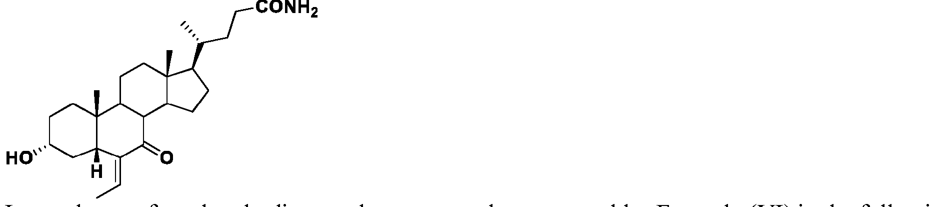

In another preferred embodiment, the compound represented by Formula (VI) is the following compound:

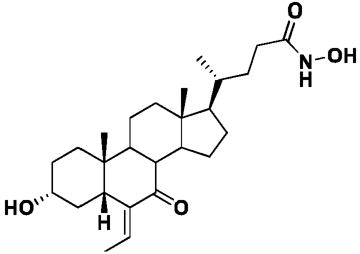

In another preferred embodiment, the compound of Formula (A) is selected from:

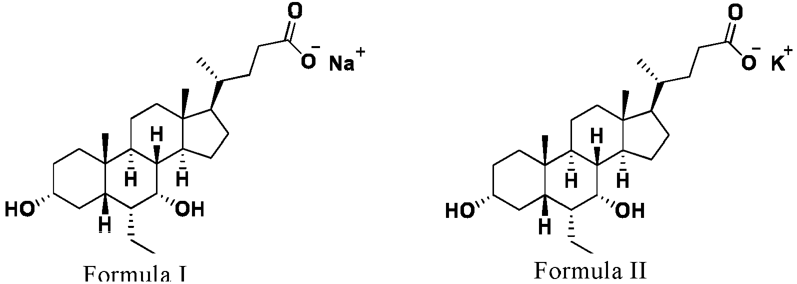

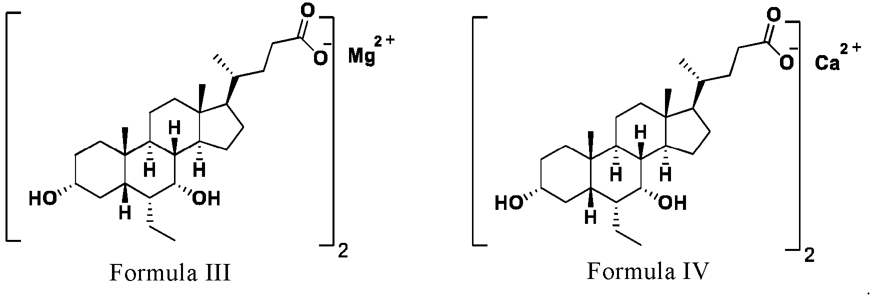

In another preferred embodiment, the obtained compounds of Formula I to IV have high purity, preferably have purity greater than 99.0%, further preferably have purity greater than 99.5%, and particularly preferably have purity greater than 99.7%.

In another preferred embodiment, the obtained compound of Formula I to IV contains less than 0.1% of related dimer substance Formula (B), particularly preferably less than 0.05%.

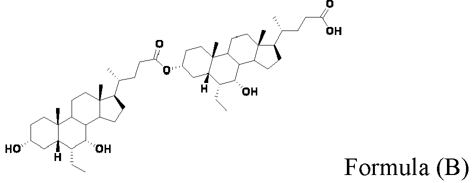

In another preferred embodiment, the obtained compound of Formula I to IV contains less than 0.1% of related isomer substance Formula (C), more preferably less than 0.05%, and particularly preferably not detected.

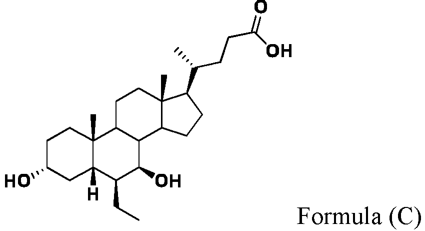

In another preferred embodiment, in the step (a), the inert solvent is selected from the group consisting of C1-C4 alcohols, water, or the combinations thereof.

In another preferred embodiment, in the step (a), the alcohol is selected from the group consisting of methanol, ethanol, isopropanol, tert-butanol, or the combinations thereof.

In another preferred embodiment, in the step (a), the inert solvent is selected from the group consisting of anhydrous methanol, or the mixed solvent of methanol:water (v/v)=1:100-100:1, preferably methanol:water (v/v)=0.5:1-5:1.

In another preferred embodiment, in the step (a), the catalytic hydrogenation is carried out in the presence of a palladium/carbon catalyst under hydrogen atomsphere.

In another preferred embodiment, the step (a) includes: in anhydrous methanol, the compound of Formula (VI) is used for catalytic hydrogenation, and then water and metal hydride are added to react to obtain a compound of Formula (V).

In another preferred embodiment, the reaction temperature in step (a) is 5 to 150°C, preferably 30 to 120°C, particularly preferably 50 to 100°C.

In another preferred embodiment, the pressure of catalytic hydrogenation in step (a) is 101,325-2,026,500 Pa (1-20 atm), preferably 202,650-2,026,500 Pa (2-20 atm).

In another preferred embodiment, in the step (a), the metal hydride reducing agent includes borohydrides, lithium *tri*-*tert*-butoxyaluminum hydride, preferably borohydrides, more preferably selected from sodium borohydride and potassium borohydride, particularly preferably sodium borohydride.

In another preferred embodiment, in the step (a), the molar ratio of the metal hydride to the compound of Formula (VI) is 5:1 to 1:1, more preferably 3:1 to 1:1, particularly preferably 2:1.

In another preferred embodiment, in the step (a), the reduction of the metal hydride is carried out under alkaline conditions. Preferably, the alkaline condition is in the presence of sodium hydroxide or potassium hydroxide.

In another preferred embodiment, the molar ratio of the added amount of sodium hydroxide or potassium hydroxide to the compound of Formula (VI) is 50:1 to 1:5, more preferably 30:1 to 10:1.

In another preferred embodiment, the method further comprises: preparing the compound of Formula (VI) by the following method:
a) condensing the compound of Formula (VII) with compound 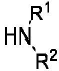 or its hydrochloride to obtain a compound of Formula (VI); 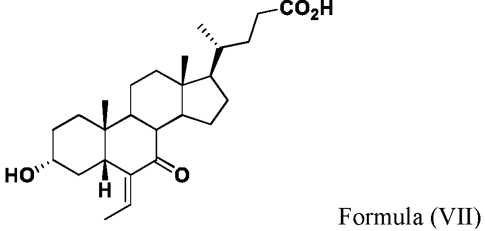 or
b) in the presence of Lewis acid, subjecting the compound of Formula (VIII) to Aldol condensation with acetaldehyde to obtain the compound of Formula (VI); 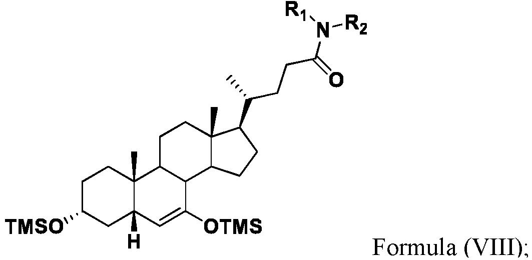 wherein, the definition of each group is as described in the first aspect of the invention. In another preferred embodiment, 1 2 HNR R or its hydrochloride is selected from the group consisting of ammonium chloride, hydroxylamine hydrochloride or combinations thereof, preferably ammonium chloride.

In another preferred embodiment, in the step a), the molar ratio of the amount of the compound 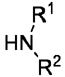 added to the compound of Formula (VII) is 10:1 to 1:1, more preferably 5:1 to 1:1, particularly preferably 2:1~1:1.

In another preferred embodiment, the condensing agent in the step a) is selected from the group consisting of N,N'-carbonyldiimidazole (CDI), EDCl, DIC, DCC, HATU, HBTU, TBTU and PyBOP; preferably HATU, HBTU and PyBOP; particularly preferably PyBOP.

In another preferred embodiment, in the step a), the molar ratio of the added amount of the condensing agent to the compound of Formula (VII) is 10:1 to 1:5, more preferably 5:1 to 1:1, particularly preferably 2:1.

In another preferred embodiment, the step a) also includes the step of adding activator, and the activator is selected from the group consisting of DMAP, HOBt, 4-PPY, DIPEA and Et₃N; preferably DIPEA.

In another preferred embodiment, the step a) is carried out in an aprotic solvent; preferably, the aprotic solvent is selected from the group consisting of dichloromethane, acetonitrile, N,N-dimethylmethyl amide, dimethyl sulfoxide, or the combinations thereof; preferably N,N-dimethylformamide.

In another preferred embodiment, the step b) is carried out in a solvent, and the solvent is selected from the group consisting of dichloromethane, acetonitrile, dimethyl sulfoxide, tetrahydrofuran, 1,4-dioxane, or the combinations thereof; preferably dichloromethane.

In another preferred embodiment, the Lewis acid in the step b) is selected from the group consisting of hydrochloric acid, acetic acid, *p*-toluenesulfonic acid, boron trifluoride ether solution, boron trifluoride acetonitrile solution, or the combinations thereof; preferably boron trifluoride ether solution.

In another preferred embodiment, in the step b), the molar ratio of the added amount of the Lewis acid to the compound of Formula (VIII) is 10:1 to 1:3, more preferably 4:1 to 2:1.

In another preferred embodiment, the method further comprises the step: preparing the compound of Formula (VIII) by the following method:
c) the compound of Formula (IX) is condensed with compound 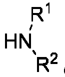 or its hydrochloride 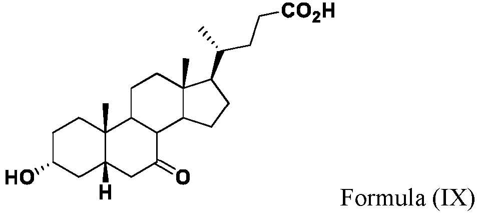 to obtain a compound of Formula (X); 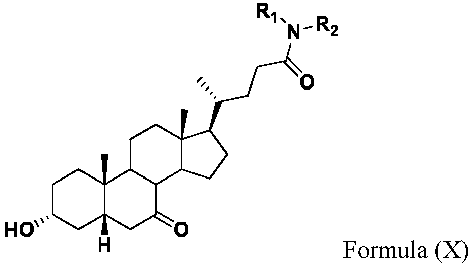
d) in the presence of a base, treating the compound of Formula (X) with trimethylchlorosilane to obtain the compound of Formula (VIII);
wherein, the definition of each group is as described in the first aspect of the invention.

In another preferred embodiment, in the step c), the condensing agent is selected from the group consisting of N,N'-carbonyldiimidazole (CDI), EDCl, DIC, DCC, HATU, HBTU, TBTU and PyBOP, or the combinations thereof; preferably HATU, HBTU and PyBOP, or the combinations thereof; particularly preferably PyBOP.

In another preferred embodiment, in the step c), the molar ratio of the added amount of the compound 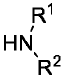 to the compound of Formula (IX) is 10:1 to 1:1, more preferably 5:1 to 1:1, particularly preferably 2:1 to 1:1.

In another preferred embodiment, in the step c), the molar ratio of the added amount of the condensing agent to the compound of Formula (IX) is 10:1 to 1:5, more preferably 5:1 to 1:1, particularly preferably 2:1.

In another preferred embodiment, the step c) also includes the step of adding activator, and the activator is selected from the group consisting of DMAP, HOBt, 4-PPY, DIPEA and Et₃N; preferably DIPEA.

In another preferred embodiment, the step c) is carried out in an aprotic solvent; preferably, the aprotic solvent is selected from the group consisting of dichloromethane, acetonitrile, N,N-dimethylmethyl amide, dimethyl sulfoxide, or the combinations thereof; preferably N,N-dimethylformamide.

In another preferred embodiment, the base in step d) is selected from the group consisting of sodium hydroxide, potassium hydroxide, sodium methoxide, sodium ethoxide, potassium *tert-*butoxide, sodium hydride, lithium diisopropylamide (LDA), or the combinations thereof; preferably lithium diisopropylamide.

In another preferred embodiment, in the step d), the molar ratio of the added amount of trimethylchlorosilane to the compound of Formula (X) is 20:1 to 1:1, more preferably 15:1 to 1:1, particularly preferably is 10:1.

In another preferred embodiment, in the step d), the molar ratio of the added amount of the base to the compound of Formula (X) is 20:1 to 1:1, more preferably 15:1 to 1:1, particularly preferably 10:1.

In another preferred embodiment, the step d) is carried out in a solvent selected from the group consisting of dichloromethane, tetrahydrofuran, ether, toluene, 1,4-dioxane, acetonitrile, N,N-dimethylformamide, dimethyl sulfoxide or the mixed solvents thereof, more preferably tetrahydrofuran.

In another preferred embodiment, the salt-forming and crystallization comprises:
(b1) mixing the 3α,7α-dihydroxy-6α-ethyl-5β-cholan-24-oic acid obtained in step (a) with pure water, adding aqueous sodium hydroxide solution, and stirring until it is substantially dissolved to obtain a clear solution;
(b2) slowly adding the aqueous solution containing Mg²⁺ or Ca²⁺ dropwise to the clear solution, and continue stirring until precipitation occurs;
(b3) flitering the precipitate, washing, and drying in vacuum to obtain the compound of Formula (A).

In another preferred embodiment, the weight ratio of the 3α,7α-dihydroxyl-6α-ethyl-5β-cholan-24-oic acid to pure water is 40:1 to 1:5, more preferably 10:1 to 1:3, particularly preferably 5:1.

In another preferred embodiment, the weight ratio of the 3α,7α-dihydroxyl-6α-ethyl-5β-cholan-24-oic acid to the sodium hydroxide aqueous solution is 10:1 to 1:10, more preferably 3:1 to 1:5, particularly preferably 1:0.97 to 1:3.

In another preferred embodiment, the concentration of the sodium hydroxide aqueous solution is 0.5-5 mol.L⁻¹, more preferably 2-3 mol.L⁻¹, particularly preferably 2.52 mol.L⁻¹.

In another preferred embodiment, the concentration of the aqueous solution containing metal ions Mg²⁺ is 0.1-10, more preferably 0.3-5, particularly preferably 0.5-2 mol·L⁻¹.

In another preferred embodiment, the concentration of the aqueous solution containing metal ions Ca²⁺ is 0.1-20, more preferably 0.5-10, particularly preferably 2-5 mol·L⁻¹.

In another preferred embodiment, the salt-forming and crystallization comprises:
(b4) dissolving the 3α,7α-dihydroxy-6α-ethyl-5β-cholan-24-oic acid obtained in step (a) in methanol, slowly dripping into the methanol solution of sodium hydroxide, and stirring until it is substantially dissolved to obtain clear solution;
(b5) concentrating the clear solution to dryness, and then adding acetone to bring to dryness so as to obtain a solid residue;
(b6) adding acetone to the residue and pulping, filtering and vacuum drying to obtain the compound of Formula (A).

### EMBODIMENTS FOR CARRYING OUT THE INVENTION

After long time and in-depth research, the inventors have obtained a method for preparing 3α,7α-dihydroxyl-6α-ethyl-5β-cholan-24-oic acid or salts thereof. The method has the characteristics such as short synthetic route, easy purification of intermediates and mild reaction conditions, and the obtained corresponding cholic acid or cholate has higher purity and better product quality, thus being suitable for pharmaceutical production. Based on the above findings, the inventor completed the present invention.

### Preparation of 3α,7α-dihydroxyl-6α-ethyl-5β-cholan-24-oic acid salt

The compounds of the Formula (A) structure of the present invention can be prepared by the following general scheme I and II:

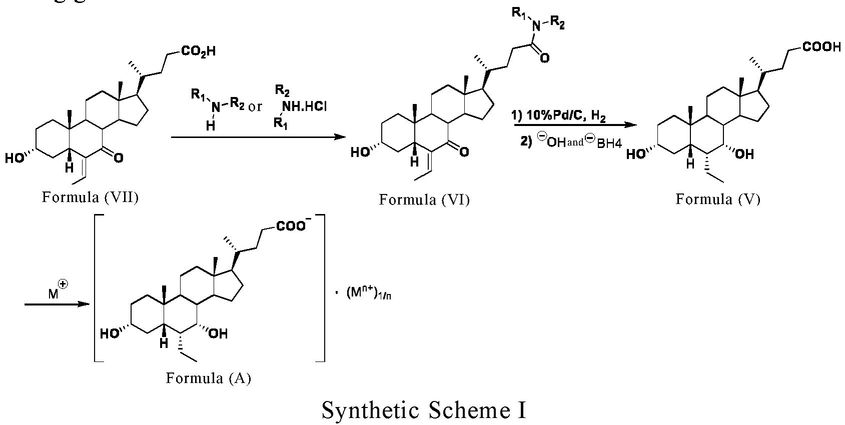

As shown in synthetic scheme I:
(1) Condensation reaction of compound of Formula (VII) and 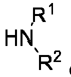 compound or its hydrochloride

The reaction is carried out in the presence of a condensing agent and/or an activator in an aprotic solvent at -20 to 60°C. The condensing agent is selected from N,N'-carbonyldiimidazole (CDI), EDCl, DIC, DCC, HATU, HBTU, TBTU and PyBOP, more preferably is PyBOP. The activator is selected from DMAP, HOBt, 4-PPY, DIPEA and Et₃N, more preferably is DIPEA; the aprotic solvent is dichloromethane, acetonitrile, N,N-dimethylformamide, dimethyl sulfoxide, or the mixed solvent thereof, more preferably is N,N-dimethylformamide; the reaction temperature is preferably -5~50°C, more preferably is 0-30°C.

(2) Obtaining the compound of Formula (V) by hydrogenating the compound of Formula (VI) and reducing with metal hydride reducing agent through the "one-pot method" to
The reaction in this step is carried out in a protic solvent, and existance of an alkaline aqueous solution at 5 to 150°C. The hydrogenation reaction uses palladium/carbon as a catalyst under 101,325-2,026,500 Pa (1-20 atm) of hydrogen; the metal hydride reducing agent can be sodium borohydride or potassium borohydride, more preferably is sodium borohydride; the protic solvent is selected from methanol, ethanol, isopropanol, *tert*-butanol, water or the mixtures thereof, preferably is the mixture of methanol and water; the alkaline aqueous solution is selected from sodium hydroxide aqueous solution and potassium hydroxide aqueous solution. The reaction temperature is 5 to 150°C, more preferably 50 to 100°C.

In a preferred embodiment, the compound of Formula (A) is prepared by the following method:

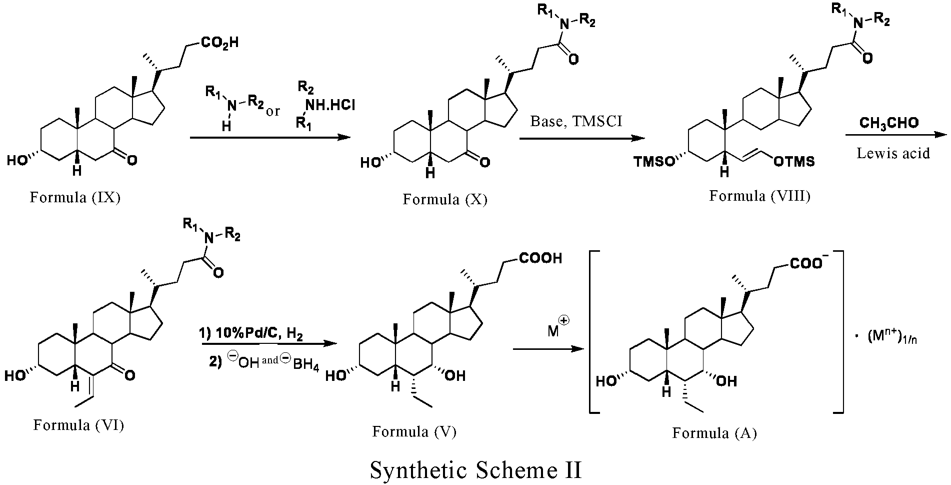

As shown in synthetic scheme II:
(1) Condensation reaction of compound of Formula (IX) and compound 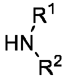 or its hydrochloride

The reaction is carried out in the presence of a condensing agent and/or an activator, in an aprotic solvent at -20 to 60°C. The condensing agent is N,N'-carbonyl diimidazole (CDI), EDC1, DIC, DCC, HATU, HBTU, TBTU and PyBOP, more preferably is PyBOP; the activating agent is DMAP, HOBt, 4-PPY, DIPEA and Et₃N, more preferably is DIPEA; the aprotic solvent is dichloromethane, acetonitrile, N,N-dimethylformamide, dimethyl sulfoxide, or the mixture, more preferably is N,N-dimethyl formamide. The reaction temperature is preferably -5~40°C, more preferably is 0-30°C.

(2) Obtaining the compound of Formula (VIII) by reacting the compound of Formula (X) with trimethylchlorosilane with the presence of base to

The reaction can be carried out in an aprotic solvent, and the base is selected from sodium hydroxide, potassium hydroxide, sodium methoxide, sodium ethoxide, potassium *tert*-butoxide, sodium hydride or lithium diisopropylamide (LDA), more preferably is lithium diisopropylamide (LDA); the aprotic solvent is selected from dichloromethane, tetrahydrofuran, ether, toluene, 1,4-dioxane, acetonitrile, N,N-dimethylformamide, dimethyl sulfoxide or the mixtures thereof, more preferably is tetrahydrofuran. The reaction temperature is preferably -100 to 40°C, more preferably -70 to 30°C. The compound of Formula (VIII) can be directly used in the next reaction without further purification.

(3) Obtaining the compound of Formula (VI) by reacting the compound of Formula (VIII) with acetaldehyde with the presence of Lewis acid

In this step, the Lewis acid is preferably boron trifluoride etherate; the solvent is selected from dichloromethane, acetonitrile, dimethyl sulfoxide, tetrahydrofuran, 1,4-dioxane, or the mixtures thereof; more preferably is dichloromethane. The reaction temperature is preferably -100 to 70°C, more preferably is -70 to 40°C.

(4) Obtaining the compound of Formula (V) by hydrogenating the compound of Formula (VI) and reducing with metal hydride reducing agent in the "one-pot method" The reaction in this step is carried out in a protic solvent, an alkaline aqueous solution at 5 to 150°C. The hydrogenation reaction use palladium/carbon as a catalyst under 101,325-2,026,500 Pa (1-20 atm) of hydrogen; the metal hydride reducing agent can be sodium borohydride or potassium borohydride, more preferably is sodium borohydride. The protic solvent is selected from methanol, ethanol, isopropanol, *tert*-butanol, water or the mixtures thereof, preferably is the mixture of methanol and water. The alkaline aqueous solution is selected from sodium hydroxide aqueous solution and potassium hydroxide aqueous solution. The reaction temperature is 5 to 150°C, more preferably 50 to 100°C.

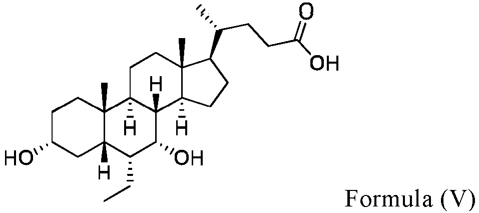

### Compared with the prior art, the preparation method of the present invention has a series of advantages. Its main advantages comprise:

(1) The intermediates obtained by amide condensation of the present invention are all in solid form, which is easy to purify, pack and store.
(2) Compared with the preparation technology in prior art, the scheme of the present invention is shorter.
(3) Compared with the preparation technology in prior art, the present invention adopts the "one-pot method" process, which has improved production continuity and is more suitable for large-scale production.
(4) Compared with the disclosed preparation technology, the corresponding cholate obtained by the present invention has higher purity and better product quality, especially, the magnesium salt, calcium salt, sodium salt and potassium salt of obeticholic acid prepared by the method of the present application has less impurity and is suitable for pharmaceutical production.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

Of the following examples, examples 1, 3, 5 and 7-10 are examples of the present invention and examples 2 and 4 are for comparison only.

### Example 1: Preparation of 3α,7α-dihydroxyl-6α-ethyl-5β-cholan-24-oic acid (Compound 1, compound of Formula (V))

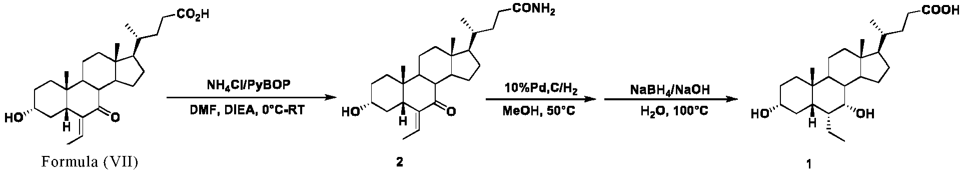

### 1. Preparation of 3α-hydroxyl-6-ethidene-7-keto-5β-chole-24-alkamide (Compound 2)

Into the flask were added 3α-hydroxyl-6-ethidene-7-keto-5β-cholan-24-oic acid (Formula (VII), 60.0g, 0.144mol), PyBOP (90.0g, 0.173mol) and N, N-dimethylformamide (400ml) were added successivly; the mixture was stirred under ice bath and cooled to 0°C. DIPEA (74.4g, 0.576mol) was added, and stirred for 30 min at 0°C. Under the protection of nitrogen, ammonium chloride (12.3g, 0.230mol) and N,N-dimethylformamide (100ml) were added. The mixture was warmed to room temperature and stirred overnight. Under stirring, the reaction mixture was slowly poured into 4L of 5% sodium bicarbonate aqueous solution, and solids were precipitated, stirred for 2 h and filtered, washed with pure water (500ml), and dried under vacuum to obtain a crude product. Ethyl acetate (360ml) was added to the crude product, refluxed and pulped for 1.0 h, then slowly cooled to about 40°C, filtered, and the filter cake was washed with ethyl acetate (50ml), and dried under vacuum to obtain the title compound (54.8g, yield 91%) , HPLC purity 98.4%. ¹H NMR (400MHz, DMSO-*d*₆) δ: 7.23 (s, 1H), 6.66 (s, 1H), 5.98 (q, J=7.2Hz, 14Hz, 1H), 4.55(d, J=4.8Hz, 1H), 3.47-3.44 (m, 1H), 2.61-2.57 (dd, J=4.0Hz, 13.2Hz, 1H), 2.32-2.17 (m, 2H), 2.11-2.04 (m, 1H), 1.98-1.79 (m, 5H), 1.70-1.57 (m, 5H), 1.44-1.05 (m, 13H), 0.95 (s, 3H), 0.90 (d, J=6.4Hz, 3H), 0.60 (s, 3H). LC-MS: 416 (M+H)⁺.

### 2. Preparation of 3α,7α-dihydroxyl-6α-ethyl-5β-cholan-24-oic acid (Compound 1)

3α-Hydroxyl-6-ethidene-7-keto-5β-chole-24-alkamide (Compound 2, 24.0g, 57.7mmol) and anhydrous methanol (130ml) were added into the flask, 10% palladium on carbon catalyst (2.5g) was added under stirring. The system was fully replaced by hydrogen, pressurized with hydrogen balloon, and reacted at 50°C for 16 h. The reaction solution was transferred to another flask, purified water (300ml) and sodium hydroxide (58g) were added under stirring, and stirred at 100°C for about 2h; filtered, and the mother liquor was transferred to another reaction flask. Sodium borohydride (5.5 g) was slowly added in batches, and the mixture was stirred in an oil bath at 100°C for about 4 h after that. The solution was concentrated under reduced pressure to remove the organic solvent, cooled in an ice bath, and 6N hydrochloric acid was slowly added dropwise with stirring to adjust the pH to about 3, extracted with ethyl acetate (450ml × 2), the organic phase was washed with water (600ml) and saturated brine (600ml) respectively, dried, and concentrated to obtain a crude solid. The title compound (21.1g, yield 85%) was obtained by recrystallization from mixture of butyl acetate/n-heptane (75ml:30ml) with HPLC purity of 99.5%. ¹H NMR (400MHz, DMSO-d6) δ: 11.97 (brs, 1H), 4.31 (s, 1H), 4.06 (d, J=5.2Hz, 1H), 3.50 (s, 1H), 3.17-3.06 (m, 1H), 2.28-2.20 (m, 1H), 2.15-2.07 (m, 1H), 1.93-1.90 (m, 1H), 1.83-1.67 (m, 6H), 1.54-1.0 (m, 18H), 0.90-0.82 (m, 9H), 0.61 (s, 3H). LC-MS: 419 (M-H)⁻, 839 (2M-H)⁻.

### Example 2: Preparation of 3α,7α-dihydroxyl-6α-ethyl-5β-cholan-24-oic acid (Compound 1)

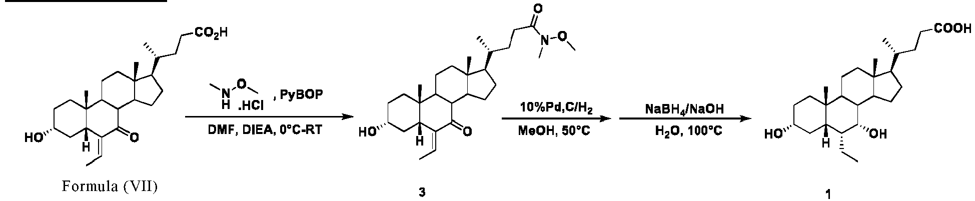

### 1. Preparation of 3α-hydroxyl-6-ethidene-7-keto-5β-chole-24-(N-methyl-N-methoxy)alkamide (Compound 3)

Into the flask were added 3α-hydroxyl-6-ethidene-7-keto-5β-cholan-24-oic acid (Formula (VII), 60.0g, 0.144mol), PyBOP (90.0g, 0.173mol) and N, N-dimethylformamide (400ml) successivly; the mixture was stirred under ice bath, cooled to 0°C, and DIPEA (74.4g, 0.576mol) was added, then stirred at 0°C for 30 min. Under the protection of nitrogen, N,O-dimethyl hydroxylamine hydrochloride (128.4g, 0.288mol) and N,N-dimethylformamide (100ml) were added. The mixture was warmed to room temperature and stirred overnight. Under stirring, the reaction mixture was slowly poured into water (4L), extracted with ethyl acetate (1L × 3), and then the organic phase was washed with saturated sodium bicarbonate solution (2L × 3), water (2L) and brine (2L), and dried over anhydrous sodium sulfate. The mixture was and concentrated to obtain a thick crude product. Ethyl acetate/petroleum ether (220ml:700ml) was added to the crude product to recrystallize, then refluxed and slowly cooled to about 0°C. The solution was filtered, and the filter cake was washed with ethyl acetate/petroleum ether (50ml × 3, EA/PE=1:4). After dried under vacuum, the title compound (51.3g, yield 78%) was obtained with HPLC purity of 98%. ¹H NMR (400MHz, DMSO-d₆) δ: 5.98 (q, J=7.2Hz, 14Hz, 1H), 4.56 (d, J=4.8Hz, 1H), 3.66 (s, 3H), 3.48-3.43 (m, 1H), 3.08 (s, 3H), 2.61-2.57 (dd, J=4.0Hz, 12.8Hz, 1H), 2.42-2.17 (m, 4H), 1.98-1.79 (m, 4H), 1.67-1.07 (m, 18H), 0.95 (s, 3H), 0.91 (d, J=6.4Hz, 3H), 0.61 (s, 3H); LC-MS: 460 (M+H)⁺.

### 2. Preparation of 3α,7α-dihydroxyl-6α-ethyl-5β-cholan-24-oic acid (Compound 1)

3α-Hydroxyl-6-ethidene-7-keto-5β-chole-24-(N-methyl-N-methoxy)alkamide (Compound 3, 5g, 10.88mmol) and anhydrous methanol (50ml) were added into the flask, 10% palladium on carbon catalyst (2.5g) were added under stirring, fully replaced by hydrogen, pressurized with the hydrogen balloon, and reacted at 50°C for 16 h. After filtration, the mother liquor was transferred to another flask, purified water (60ml) and sodium hydroxide (13.1g) were added with stirring, and the mixture was stirred at 100°C until the mixture was clear. Sodium borohydride (1.2 g) was slowly added in batches. After addition, the mixture was stirred in oil bath at 100°C for about 4 h, and concentrated under reduced pressure to remove the organic solvent. Then the residue was cooled in ice bath, 6N hydrochloric acid was slowly added dropwise under stirring to adjust the pH to about 3, and extracted with ethyl acetate (100ml × 2). The organic phase was washed with water (150ml) and saturated brine (150ml), dried, and concentrated to obtain crude solid. The title compound (2.6g, yield 56%) was obtained by crystallization from butyl acetate (13ml), of which the HPLC purity was greater than 99.0%.

### Example 3 Preparation of 3α,7α-dihydroxyl-6α-ethyl-5β-cholan-24-oic acid (Compound 1)

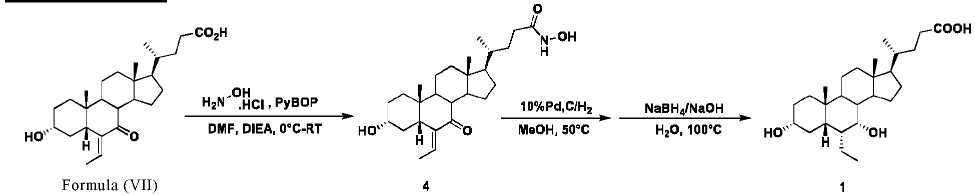

### 1. Preparation of 3α-hydroxyl-6-ethidene-7-keto-5β-chole-24-hydroxyamide (Compound 4)

Into the flask were added 3α-hydroxyl-6-ethidene-7-keto-5β-cholan-24-oic acid (Formula (VII), 300mg, 0.72mmol), PyBOP (452mg, 0.86mmol) and N, N- dimethylformamide (6ml) successivly. The mixture was stirred under ice bath, cooled to 0°C, DIPEA (372mg, 2.88mmol) was added and stirring for 30 min at 0°C. Under nitrogen protection, hydroxylamine hydrochloride (77mg, 1.08mmol) was added. The mixture was warmed to room temperature and stirred for 3h. Under stirring, the reaction mixture was slowly poured into water (60 ml) and stirred for 30 minutes. The solution was filtered, and the filter cake was washed with water (10 ml × 4), and dried under vacuum to obtain the title compound (275 mg, yield 95%), of which the HPLC purity was 98.5%. ¹H NMR (400MHz, DMSO-d₆) δ: 10.33 (d, J=1.2Hz, 1H), 8.66 (d, J=1.6Hz, 1H), 5.98 (q, J=7.2Hz, 14Hz, 1H), 4.54 (d, J=8.8Hz, 1H), 3.48-3.42 (m, 1H), 2.59 (dd, J=4.0Hz, 12.8Hz, 1H), 2.32-2.18 (m, 2H), 2.0-1.79 (m, 6H), 1.67-1.57 (m, 5H), 1.43-1.31 (m, 5H), 1.23-1.04 (m, 8H), 0.95 (s, 3H), 0.90 (d, J=6.4Hz, 3H), 0.60 (s, 3H); LC-MS: 432 (M+H)⁺.

### 2. Preparation of 3α,7α-dihydroxyl-6α-ethyl-5β-cholan-24-oic acid (Compound 1)

3α-Hydroxyl-6-ethidene-7-keto-5β-chole-24-hydroxyalkamide (Compound **4**, 0.2g, 0.46mmol) and anhydrous methanol (10ml) were added into the flask, 10% palladium on carbon catalyst (20 mg) were added under stirring. The system was fully replaced by hydrogen, pressurized with hydrogen balloon, and reacted at 50°C for 16 h. After filtration, the mother liquor was transferred to another flask, purified water (10ml) and sodium hydroxide (0.55g) were added under stirring, and the mixture was stirred at 100°C until the mixture was clear. Sodium borohydride (0.07 g) was slowly added in batches, then the mixture was stirred in an oil bath at 100°C for about 4 h. The organic solvent was removed by concentrating under reduced pressure. The residue was cooled in ice bath, 6N hydrochloric acid was slowly added dropwise with stirring to adjust the pH to about 3, and extracted with ethyl acetate (15ml × 3). The organic phase was washed with water (30ml) and saturated brine (30ml), dried and concentrated to obtain crude solid. The title compound (0.17 g, yield 87%) was obtained by crystallizing with butyl acetate (5 ml), of which the HPLC purity was greater than 99.5%.

### Example 4 Preparation of 3α,7α-dihydroxyl-6α-ethyl-5β-cholan-24-oic acid (Compound 1)

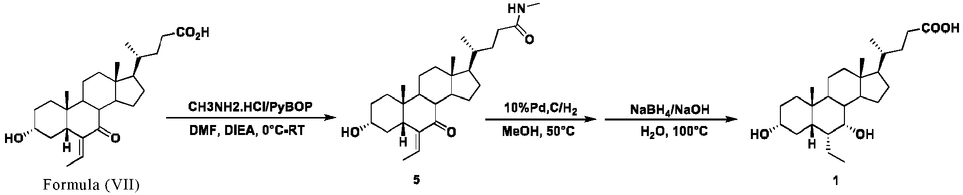

### 1. Preparation of 3α-hydroxyl-6-ethidene-7-keto-5β-chole-24-methylamide (Compound 5)

Into the flask were added 3α-hydroxyl-6-ethidene-7-keto-5β-cholan-24-oic acid (Formula (VII), 6.0g, 14.4mmol), PyBOP (9.0g, 17.3mol) and N, N-dimethylformamide (40ml) successivly. The mixture was stirred under ice bath, cooled to 0°C, DIPEA (7.5g, 57.6mol) was added, then stirred at 0°C for 30 min. Under nitrogen protection, methylamine hydrochloride (1.6g, 23.0mol) and N,N-dimethylformamide (10ml) were added. The mixture was warmed to room temperature and stirred for 3h. Under stirring, the reaction mixture was slowly poured into 5% sodium bicarbonate aqueous solution (400 ml), stirred and filtered. The filter cake was washed with water (50ml × 4), and dried under vacuum to obtain the title compound (5.8g, yield 93%). HPLC purity was 98.4%. ¹H NMR (400MHz, DMSO-d₆) δ: 7.81 (d, J=4.4Hz, 1H), 5.88 (q, J=7.2Hz, 14Hz, 1H), 4.61(d, J=4.8Hz, 1H), 3.47-3.42 (m, 1H), 2.60-2.56 (dd, J=4.0Hz, 13.2Hz, 1H), 2.54 (d, J=4.4Hz, 3H), 2.29-1.78 (m, 8H), 1.66-1.04 (m, 18H), 0.95 (s, 3H), 0.89 (d, J=6.4Hz, 3H), 0.60 (s, 3H); LC-MS: 430 (M+H)⁺.

### 2. Preparation of 3α,7α-dihydroxyl-6α-ethyl-5β-cholan-24-oic acid (Compound 1)

3α-Hydroxyl-6-ethidene-7-keto-5β-chole-24-carboxalkamide (Compound **5**, 1.0g, 2.32mmol) and anhydrous methanol (10ml) were added into the flask, 10% palladium on carbon catalyst (100 mg) were added under stirring. The system was fully replaced by hydrogen, pressurized with the hydrogen balloon, and reacted at 50°C for 16 h. After filtration, the mother liquor was transferred to another flask, purified water (120ml) and sodium hydroxide (2.8g) were added with stirring, and the mixture was stirred at 100°C until the mixture was clear. Sodium borohydride (0.27 g) was slowly added in batches. After addition, the mixture was stirred in oil bath at 100°C for about 4 h, and concentrated under reduced pressure to remove the organic solvent. The residue was cooled in an ice bath, 6N hydrochloric acid was slowly added dropwise under stirring to adjust the pH to about 3, and extracted with ethyl acetate (30ml × 3). The organic phase was washed with water (40ml) and saturated brine (40ml), dried, and concentrated to obtain a crude solid. The title compound (0.78 g, yield 80%) was obtained by crystallizing with butyl acetate (10 ml), of which the HPLC purity was greater than 99.0%.

### Example 5 Preparation of 3α,7α-dihydroxyl-6α-ethyl-5β-cholan-24-oic acid (Compound 1)

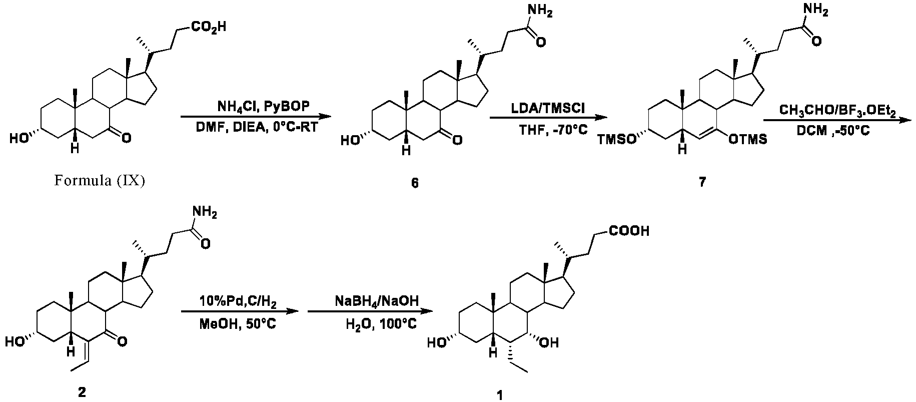

### 1. Preparation of 3α-hydroxyl-7-keto-5β-chole-24-alkamide (Compound 6)

Into the reaction flask were added 3α-hydroxyl-7-keto-5β-cholin-24-alkanoic acid (Formula (IX), 100.0g, 0.26mol), PyBOP (160.0g, 0.31mol) and N,N-dimethyl formamide (700ml); the mixture was stirred in an ice bath to about 0°C, DIPEA (116g, 0.91mol) was added, cooled to about 0°C, and stirred for 30 minutes. Under the protection of nitrogen, ammonium chloride (27.4g, 0.52mol) and N,N-dimethylformamide (100ml) were added, and the mixture was heated to room temperature and stirred for 16h. The mixture was stirred at 100°C for 16 hours. Under stirring, the reaction mixture was slowly poured into 5% sodium bicarbonate aqueous solution (5L), the solid was precipitated, and stirred evenly for 2h. The mixture was filtered, washed with pure water (500ml × 8), and dried under vacuum to obtain crude product. The crude product was transferred to a flask, added with tetrahydrofuran (400ml), refluxed and pulped. After cooled down and filtered, the residue was washed with tetrahydrofuran (50ml×3) and dried under vacuum to obtain the target compound (83.5g, 86%). ¹H NMR (400MHz, DMSO-d₆) δ: 7.23 (s, 1H), 6.66 (s, 1H), 4.50(d, J=5.2Hz, 1H), 3.36-3.34 (m, 1H), 2.94-2.89 (m, 1H), 2.45 (t, J=11.2Hz, 1H), 2.11-2.04 (m, 2H), 1.98-1.92 (m, 2H), 1.85-1.66 (m, 6H), 1.51-0.92 (m, 17H), 0.89 (d, J=6.4Hz, 3H), 0.62 (s, 3H); ESI-MS (m/z): 390 (M+H)⁺.

### 2. Preparation of 3α,7-bis(trimethylsiloxy)-5β-chole-6-en-24-alkamide (Compound 7)

Dry tetrahydrofuran (200ml) and lithium diisopropylamide (193ml, 2M heptane/tetrahydrofuran/ethylbenzene solution) were added to the flask successively, cooled to -70°C under nitrogen, and stirred for 20 minutes. Trimethyl chlorosilane (42g, 0.385mol) was slowly dropped, and continued to stir for 30min at -70°C. The suspension of 3α-hydroxyl-7-keto-5β-chole-24-alkamide (Compound **6**) in tetrahydrofuran (15.0g solids dispersed in 50ml dry tetrahydrofuran uniformly) was dropped into the mixture in about 10 minutes, and continued to stir for 1 hour at about -70°C. After cooled in an ice bath, the reaction mixture was slowly added to saturated sodium bicarbonate solution to quench, and extracted with ethyl acetate (150ml × 2). The organic phase was washed with saturated sodium bicarbonate solution (200ml × 3) for several times, and finally washed with brine, dried and concentrated to obtain the crude product, which was directly used in the next reaction without purification. ¹H NMR (400MHz, CD₃OD) δ: 4.75 (q, J=1.6, 4.4Hz, 1H), 3.65-3.58 (m, 1H), 2.32-2.25 (m, 1H), 2.18-2.09 (m, 2H), 1.99-1.73 (m, 7H), 1.66-1.43 (m, 5H), 1.37-1.09 (m, 9H), 1.01 (d, J=6.8Hz, 3H), 0.88 (s, 3H) , 0.75 (s, 3H) , 0.18 (s, 9H) , 0.13 (s, 9H); ESI-MS (m/z): 534 (M+H)⁺.

### 3. Preparation of 3α-hydroxyl-6-ethidene-7-keto-5β-chole-24-alkamide (Compound 2)

Into the flask were added 3α,7-bis(trimethylsiloxy)-5β-chole-6-en-24-alkamide (Compound 7, 1.0g, 1.9mmol), dry dichloromethane (10ml) successivly, cooled to -60°C under nitrogen and stirred for 10 min. Acetaldehyde (230ul, 3.8mmol) was quickly added to the mixed solution, and continued stirring for 15 minutes at about -60°C. The boron trifluoride ether in dichloromethane (1.0ml of 48% boron trifluoride ether dissolved in 5ml of dry dichloromethane) was added dropwise into the mixture, and continued stirring for 20min at - 50°C, then heated to 50°C and stirred for 1h. After cooled in an ice bath, the reaction mixture was slowly poured into saturated aqueous sodium bicarbonate solution under stirring to quench. After extracted with dichloromethane (20ml × 2), the combined organic phases was washed with water and brine, dried and concentrated to give a crude product. The target product (420 mg, yield 54%) was obtained by crystallizing with mixed system of ethyl acetate and ethanol.

### 4. Preparation of 3α,7α-dihydroxyl-6α-ethyl-5β-cholan-24-oic acid (Compound 1)

The method was similar to the preparation step 2 of Compound 1 in Example 1 to obtain Compound 1, yield 82%, HPLC purity was 99.6%.

### Example 7 Preparation of 3α,7α-dihydroxyl-6α-ethyl-5β-cholan-24-oic acid magnesium salt (Compound 8)

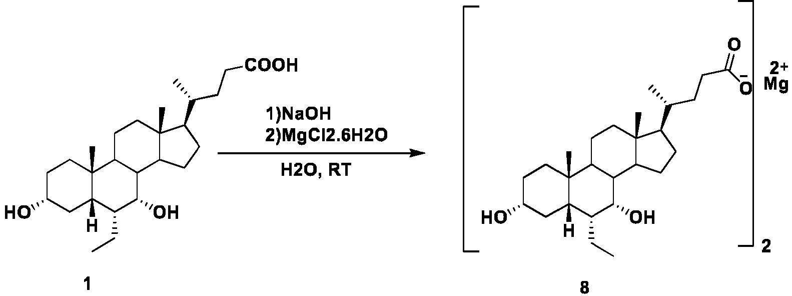

3α,7α-Dihydroxyl-6α-ethyl-5β-cholan-24-oic acid (Compound 1, 450g) and pure water (2.25kg) were added into a 10L reactor, and sodium hydroxide aqueous solution (42.3g hydrogen sodium oxide dissolved in 420g water) was added with stirring, stirred at about 35°C until it is almost clear. The mixture was filtered, washed with a small amount of water. The mother liquor was transferred to the reactor, and magnesium chloride aqueous solution (130.5g magnesium chloride hexahydrate in 969g water) was slowly added dropwise, washed with 204g pure water and transferred together, then continued to stir for 1.5 h. After filtered, the residue was washed with pure water to neutralize, and dried under vacuum at 65°C to obtain the title compound (460 g, yield 99%), purity 99.8%, in which Formula (B) impurity was 0.02%, and Formula (C) impurity was undetected.

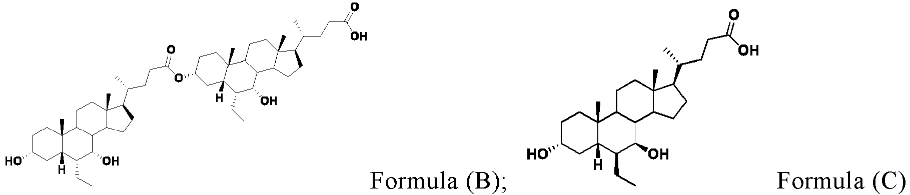

¹H NMR (400MHz, DMSO-d6) δ: 4.32 (s, 1H), 4.03 (d, J=4.0Hz, 1H), 3.50 (s, 1H), 3.13 (s, 1H), 1.93-0.81(m, 36H), 0.61 (s, 3H).

Infrared spectrum (IR) characteristic absorption peaks: 3401±5cm⁻¹, 2935±5cm⁻¹, 2871±5cm⁻¹, 1555±5cm⁻¹, 1449±5cm⁻¹, 1414±5cm⁻¹, 1377±5cm⁻¹, 1159±5cm⁻¹, 1064±5cm⁻¹ and 603±5cm⁻¹.

Atomic absorption (Mg²⁺): 2.82% (calculated value: 2.81%).

### Example 8 Preparation of 3α,7α-dihydroxyl-6α-ethyl-5β-cholin-24-sodium salt (Compound 9)

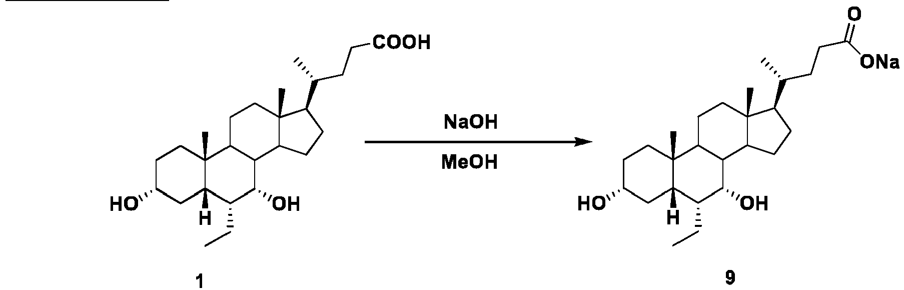

5.0g of Compound 1 was stirred with 10ml of anhydrous methanol to dissolve to clear solution, then slowly dropped into sodium hydroxide methanol solution (1.0eq NaOH dissolved in 2.0ml methanol), and stirred at room temperature for 2h. The mixture was concentrated in water bath at 40°C, then acetone (15 ml × 3) was added and concentrated to dryness. Finally, 30 ml of acetone was added to the residue to pulp for 2 hours, filtered, and dried under vacuum at room temperature for 8 hours to obtain the title compound 4.6g; yield: 87%, purity 99.7%, the Formula (B) impurity was 0.03%, and the Formula (C) impurity hasn't been detected.
¹H NMR (400MHz, DMSO-*d*₆) δ: 4.38(s, 1H), 4.08(s, 1H), 3.50(s, 1H), 3.17-3.13(m, 1H), 1.93-0.81(m, 36H), 0.61(s, 3H)

Infrared spectrum (IR) characteristic absorption peaks: 3423±5cm⁻¹, 2958±5cm⁻¹, 2935±5cm⁻¹, 2871±5cm⁻¹, 2122±5cm⁻¹, 1640±5cm⁻¹, 1559±5cm⁻¹, 1451±5cm⁻¹, 1406±5cm⁻¹, 1378±5cm⁻¹, 1160±5cm⁻¹, 1065±5cm⁻¹ and 603±5cm⁻¹.

Ion chromatography IC (Na⁺): 5.19% (calculated value: 5.19%).

### Example 9 Preparation of 3α,7α-dihydroxyl-6α-ethyl-5β-cholan-24-oic acid potassium salt (Compound 10)

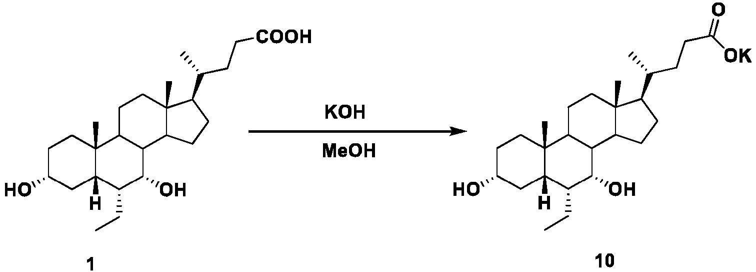

5.0g of Compound 1 was stirred with 10 ml of methanol to dissolve to clear solution, then slowly dripped into potassium hydroxide methanol solution (0.65g KOH dissolved in 2.0ml methanol) and stirred at room temperature for 2h; After concentrated to dryness in 40°C water bath, acetone (10 ml × 3 ) was added to the residue to concentrate to dryness. 30ml acetone was added to the residue and pulped for 1.5h, and filtered and dried under vacuum at room temperature for 8h to obtain the title Compound 10 4.9g; yield: 90%, purity 99.8%, Formula (B) impurity 0.03%, Formula (C) impurity hasn't been detected.

¹H NMR (400MHz, DMSO-*d*₆) δ: 4.51(s, 1H), 4.18(s, 1H), 3.50(s, 1H), 3.15-3.10(m, 1H), 1.93-0.81(m, 36H), 0.60(s, 3H).

Infrared spectrum (IR) characteristic absorption peaks: 3409±5cm⁻¹, 2935±5cm⁻¹, 2871±5cm⁻¹, 1643±5cm⁻¹, 1555±5cm⁻¹, 1465±5cm⁻¹, 1451±5cm⁻¹, 1404±5cm⁻¹, 1378±5cm⁻¹, 1338±5cm⁻¹, 1159±5cm⁻¹, 1065±5cm⁻¹ and 603±5cm⁻¹.

Ion chromatography IC (K⁺): 8.50% (calculated value: 8.52%).

### Example 10 Preparation of 3α,7α-dihydroxyl-6α-ethyl-5β-cholan-24-oic acid calcium salt (Compound 11)

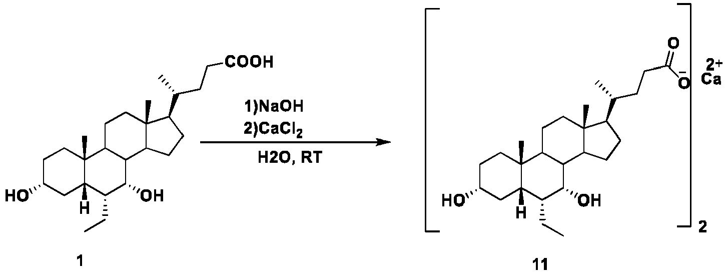

840 mg of Compound 1 was stirred with 3.0ml water and sodium hydroxide aqueous solution (85 mg solid dissolved in 2.0 ml water) to dissolve to clear; calcium chloride aqueous solution (888 mg calcium chloride solid dissolved in 2.5 ml water) was added dropwise, stirred at room temperature for 16 hours, filtered, washed with pure water and dried. The residue was dried under vacuum at room temperature for 8 hours to obtain the title compound 735mg; yield: 84%, purity 99.9%, Formula (B) impurity was 0.01%, and Formula (C) impurity hasn't been detected.

¹H NMR (400MHz, DMSO-*d*₆) δ: 4.32(s, 1H), 4.02(s, 1H), 3.50(s, 1H), 3.14(m, 1H), 2.09-0.81(m, 36H), 0.61(s, 3H).

Infrared spectrum (IR) characteristic absorption peaks: 3407±5cm⁻¹, 2935±5cm⁻¹, 2871±5cm⁻¹, 1553±5cm⁻¹, 1447±5cm⁻¹, 1417±5cm⁻¹, 1377±5cm⁻¹, 1159±5cm⁻¹, 1064±5cm⁻¹and 603±5cm⁻¹.

Atomic absorption: 4.58% (calculated value:4.56%).

## Claims

1. A method for preparing a compound represented by General Formula (A): 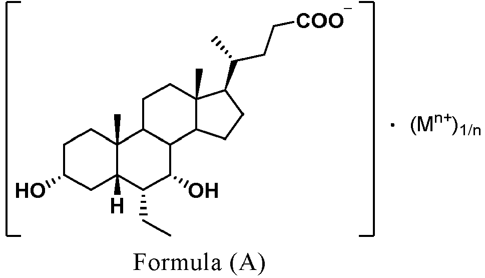
wherein, n is 1 or 2, and M is NH₄⁺, alkali metal ion, alkaline earth metal ion or transition metal ion;
the method comprises the following steps:
(a) in an inert solvent, subjecting the compound of Formula (VI) to the catalytic hydrogenation and reducing with metal hydride reducing agent by "one-pot method" 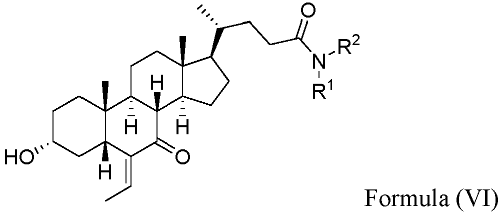 to obtain the compound of Formula (V); 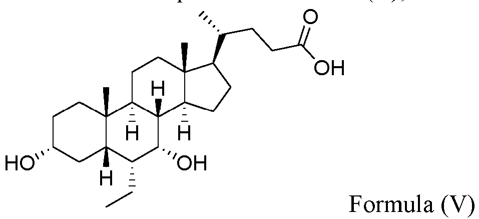
(b) salifying compound of Formula (V) and crystallizing in a solvent to obtain the compound of Formula (A);
wherein, R¹ and R² are both hydrogen, or R¹ is hydrogen, and R² is hydroxyl.

2. The method of claim 1, wherein the compound of Formula (A) is selected from:

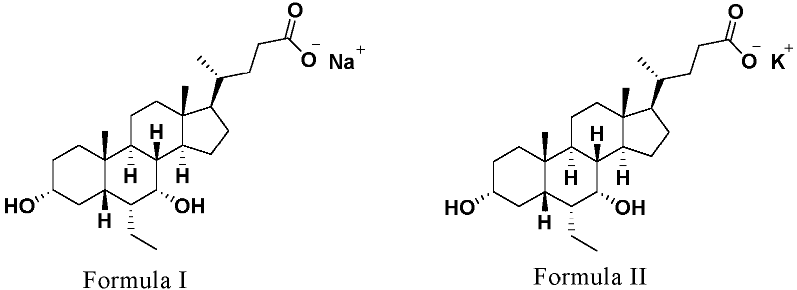

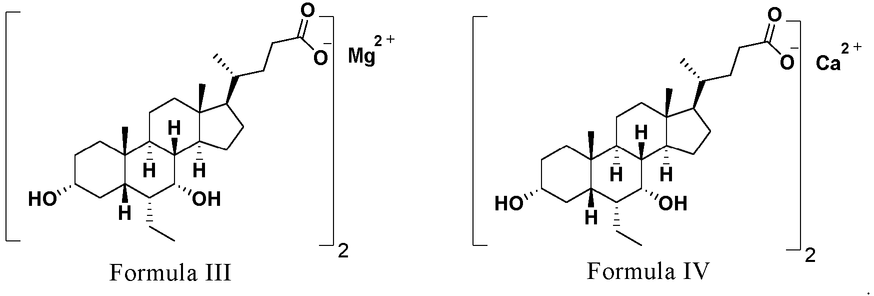

3. The method of claim 1, wherein in the step (a), the inert solvent is selected from the group consisting of C1-C4 alcohols, water, or the combinations thereof.

4. The method of claim 1, wherein in the step (a), the alcohol is selected from the group consisting of methanol, ethanol, isopropanol, *tert*-butanol, or the combinations thereof.

5. The method of claim 1, wherein in the step (a), the inert solvent is selected from the group consisting of anhydrous methanol, or the mixed solvent of methanol:water (v/v)=1:100-100:1, preferably methanol:water (v/v)=0.5:1-5:1.

6. The method of claim 1, wherein the method further comprises: preparing the compound of Formula (VI) by the following method:
a) condensing the compound of Formula (VII) with compound 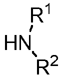 or its hydrochloride to obtain a compound of Formula (VI); 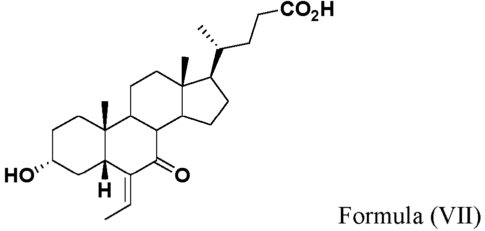 or
b) in the presence of Lewis acid, subjecting the compound of Formula (VIII) to Aldol condensation with acetaldehyde to obtain the compound of Formula (VI); 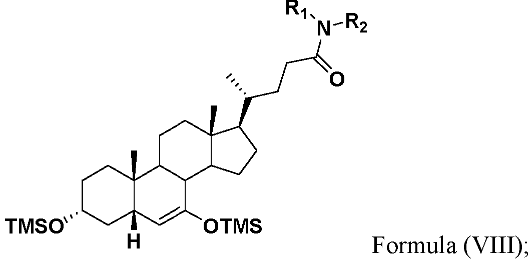 wherein, the definition of each group is as described in claim 1.

7. The method of claim 6, wherein the condensing agent in step a) is selected from the group consisting of N,N'-carbonyldiimidazole (CDI), EDCl, DIC, DCC, HATU , HBTU, TBTU and PyBOP; preferably HATU, HBTU and PyBOP; particularly preferably PyBOP.

8. The method of claim 6, wherein the method further comprises the step: preparing the compound of Formula (VIII) by the following method:
c) the compound of Formula (IX) is condensed with compound 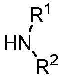 or its hydrochloride 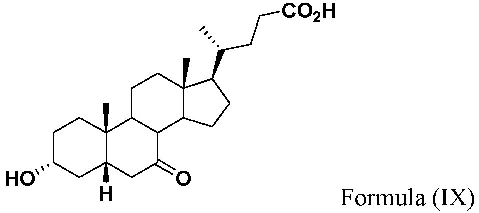 to obtain a compound of Formula (X); 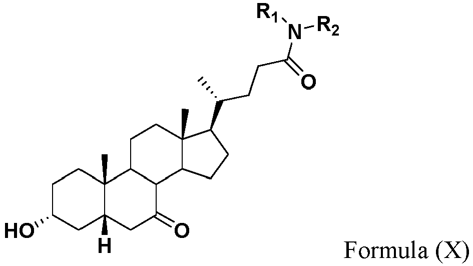
d) in the presence of a base, treating the compound of Formula (X) with trimethylchlorosilane to obtain the compound of Formula (VIII);
wherein, the definition of each group is as described in claim 1.

9. The method of claim 1, wherein the salt-forming and crystallization comprises:
(b1) mixing the 3α,7α-dihydroxy-6α-ethyl-5β-cholan-24-oic acid obtained in step (a) with pure water, adding aqueous sodium hydroxide solution, and stirring until it is substantially dissolved to obtain a clear solution;
(b2) slowly adding the aqueous solution containing Mg²⁺ or Ca²⁺ dropwise to the clear solution, and continue stirring until precipitation occurs;
(b3) flitering the precipitate, washing, and drying in vacuum to obtain the compound of Formula (A).

10. The method of claim 1, wherein the salt-forming and crystallization comprises:
(b4) dissolving the 3α,7α-dihydroxy-6α-ethyl-5β-cholan-24-oic acid obtained in step (a) in methanol, slowly dripping into the methanol solution of sodium hydroxide, and stirring until it is substantially dissolved to obtain clear solution;
(b5) concentrating the clear solution to dryness, and then adding acetone to bring to dryness so as to obtain a solid residue;
(b6) adding acetone to the residue and pulping, filtering and vacuum drying to obtain the compound of Formula (A).

## Patentansprüche

1. Verfahren zur Herstellung einer Verbindung, die durch die allgemeine Formel (A) dargestellt wird: 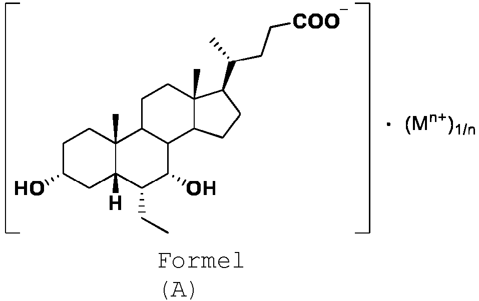
wobei n = 1 oder 2 ist und M = NH₄⁺, ein Alkalimetallion, ein Erdalkalimetallion oder ein Übergangsmetallion ist;
das Verfahren die folgenden Schritte umfasst:
(a) katalytisches Hydrieren und Reduzieren der Verbindung der Formel (VI) mit einem Metallhydrid als Reduktionsmittel in einem inerten Lösungsmittel durch ein "!Eintopfverfahren": 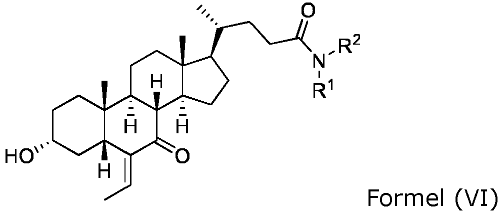 wobei man die Verbindung der Formel (V) erhält; 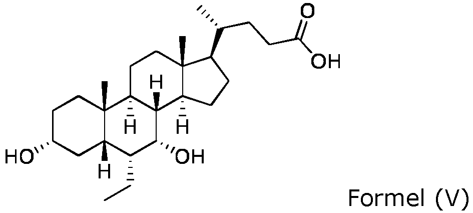
(b) Aussalzen der Verbindung der Formel (V) und Kristallisieren in einem Lösungsmittel, wobei man die Verbindung der Formel (A) erhält;
wobei R¹ und R² beide Wasserstoff sind oder R¹ Wasserstoff ist und R² Hydroxy ist.

2. Verfahren gemäß Anspruch 1, wobei die Verbindung der Formel (A) ausgewählt ist aus:

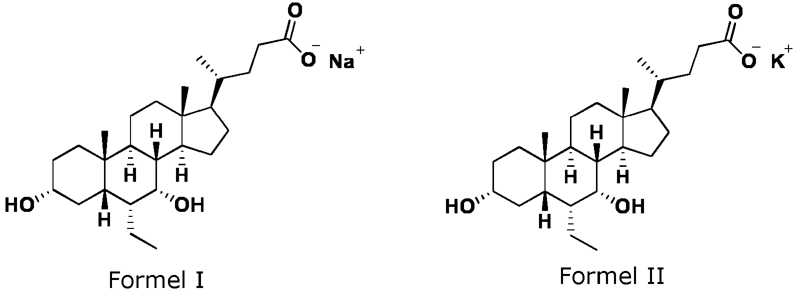

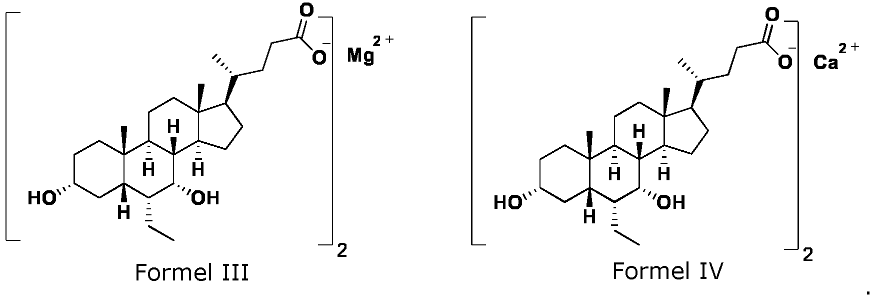

3. Verfahren gemäß Anspruch 1, wobei in Schritt (a) das inerte Lösungsmittel aus der Gruppe ausgewählt ist, die aus C₁-C₄-Alkoholen, Wasser oder Kombinationen davon besteht.

4. Verfahren gemäß Anspruch 1, wobei in Schritt (a) der Alkohol aus der Gruppe ausgewählt ist, die aus Methanol, Ethanol, Isopropanol, tert-Butanol oder Kombinationen davon besteht.

5. Verfahren gemäß Anspruch 1, wobei in Schritt (a) das inerte Lösungsmittel aus der Gruppe ausgewählt ist, die aus wasserfreiem Methanol oder dem Lösungsmittelgemisch aus Methanol : Wasser (v/v) = 1:100 bis 100:1, vorzugsweise Methanol : Wasser (v/v) = 0,5:1 bis 5:1, besteht.

6. Verfahren gemäß Anspruch 1, wobei das Verfahren weiterhin umfasst: das Herstellen der Verbindung der Formel (VI) nach dem folgenden Verfahren:
a) Kondensieren der Verbindung der Formel (VII) mit Verbindung 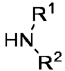 oder ihrem Hydrochlorid, wobei man eine Verbindung der Formel (VI) erhält; 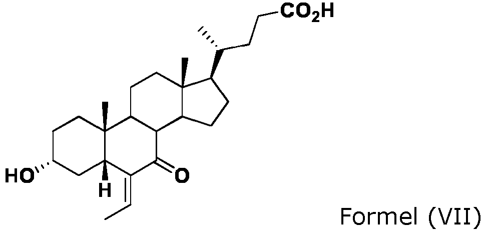 oder
b) Durchführen einer Aldolkondensation mit der Verbindung der Formel (VIII) und Acetaldehyd in Gegenwart einer Lewis-Säure, wobei man die Verbindung der Formel (VI) erhält; 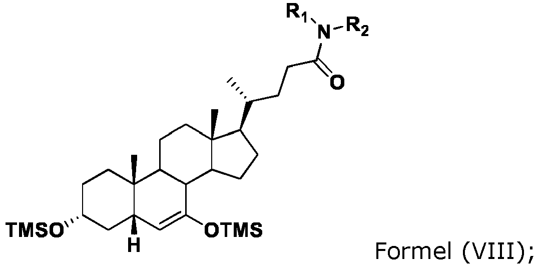 wobei die Definition jeder Gruppe wie in Anspruch 1 beschrieben ist.

7. Verfahren gemäß Anspruch 6, wobei das Kondensationsmittel in Schritt a) aus der Gruppe ausgewählt ist, die aus N,N'-Carbonyldiimidazol (CDI), EDCl, DIC, DCC, HATU , HBTU, TBTU und PyBOP; vorzugsweise HATU, HBTU und PyBOP; besonders bevorzugt PyBOP, besteht.

8. Verfahren gemäß Anspruch 6, wobei das Verfahren weiterhin den folgenden Schritt umfasst: das Herstellen der Verbindung der Formel (VIII) nach dem folgenden Verfahren:
c) das Kondensieren der Verbindung der Formel (IX) mit Verbindung 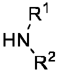 oder ihrem Hydrochlorid: 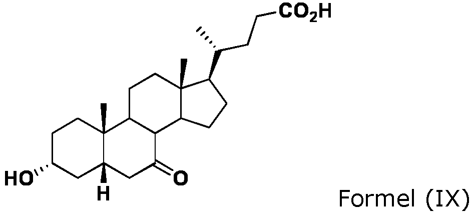 wobei man eine Verbindung der Formel (X) erhält; 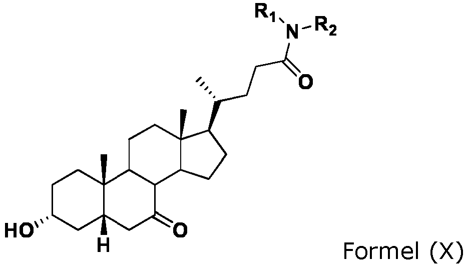
d) das Behandeln der Verbindung der Formel (X) mit Trimethylchlorsilan in Gegenwart einer Base, wobei man die Verbindung der Formel (VIII) erhält;
wobei die Definition jeder Gruppe wie in Anspruch 1 beschrieben ist.

9. Verfahren gemäß Anspruch 1, wobei die Salzbildung und Kristallisation Folgendes umfasst:
(b1) Mischen der in Schritt (a) erhaltenen 3α,7α-Dihydroxy-6α-ethyl-5β-cholan-24-säure mit reinem Wasser, Hinzufügen von Natronlauge und Rühren, bis sie im Wesentlichen aufgelöst ist und man eine klare Lösung erhält;
(b2) langsames tropfenweises Hinzufügen der wässrigen Lösung, die Mg²⁺ oder Ca²⁺ enthält, zu der klaren Lösung und Weiterrühren, bis eine Fällung erfolgt;
(b3) Abfiltrieren des Niederschlags, Waschen und Trocknen im Vakuum, wobei man die Verbindung der Formel (A) erhält.

10. Verfahren gemäß Anspruch 1, wobei die Salzbildung und Kristallisation Folgendes umfasst:
(b4) Auflösen der in Schritt (a) erhaltenen 3α,7α-Dihydroxy-6α-ethyl-5β-cholan-24-säure in Methanol, langsames Tröpfeln von Natriumhydroxid in die Methanollösung und Rühren, bis sie im Wesentlichen aufgelöst ist und man eine klare Lösung erhält;
(b5) Einengen der klaren Lösung bis zur Trockne und dann Hinzufügen von Aceton, um Trockenheit zu erreichen, so dass man einen festen Rückstand erhält;
(b6) Hinzufügen von Aceton zu dem Rückstand und Zerdrücken, Filtrieren und Vakuumtrocknen, wobei man die Verbindung der Formel (A) erhält.

## Revendications

1. Procédé de préparation d'un composé représenté par la Formule Générale (A) : 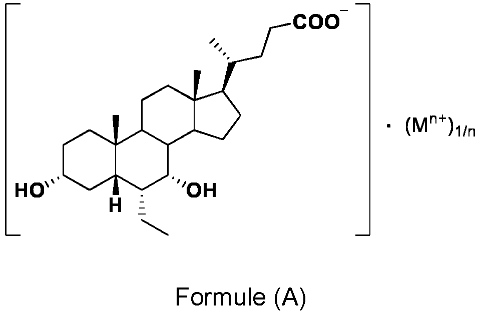
dans laquelle n vaut 1 ou 2, et M est NH₄⁺, un ion de métal alcalin, un ion de métal alcalino-terreux ou un ion de métal de transition ;
le procédé comprend les étapes suivantes :
(a) dans un solvant inerte, soumettre le composé de formule (VI) à l'hydrogénation catalytique et le réduire à l'aide d'un agent réducteur à base d'hydrure métallique par un procédé monotope 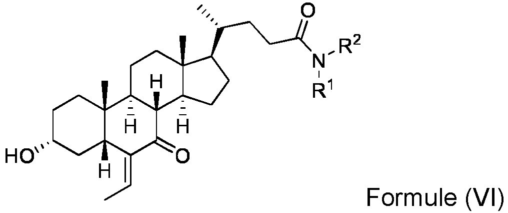 pour obtenir le composé de Formule (V) ; 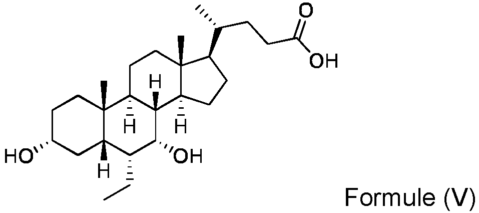
(b) salification du composé de Formule (V) et cristallisation dans un solvant pour obtenir le composé de Formule (A) ;
où R¹ et R² sont tous deux hydrogène, ou R¹ est hydrogène, et R² est hydroxyle.

2. Procédé selon la revendication 1, dans lequel le composé de Formule (A) est choisi parmi :

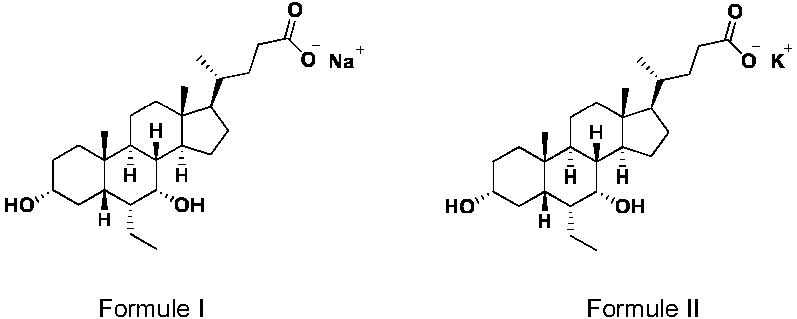

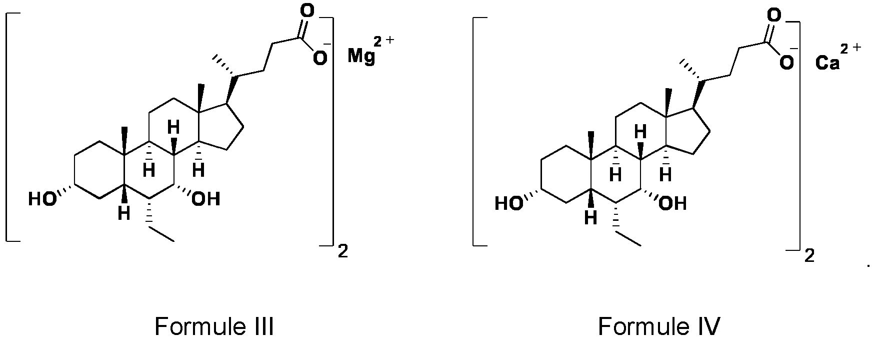

3. Procédé selon la revendication 1, dans lequel, à l'étape (a), le solvant inerte est choisi dans le groupe constitué d'alcools en C1-C4, d'eau, ou de combinaisons de ceux-ci.

4. Procédé selon la revendication 1, dans lequel, à l'étape (a), l'alcool est choisi dans le groupe constitué de méthanol, d'éthanol, d'isopropanol, de *tert-*butanol, ou de combinaisons de ceux-ci.

5. Procédé selon la revendication 1, dans lequel, à l'étape (a), le solvant inerte est choisi dans le groupe constitué de méthanol anhydre, ou de solvant mixte méthanol:eau (v/v) = 1:100-100:1, de préférence de méthanol:eau (v/v) = 0,5:1-5:1.

6. Procédé selon la revendication 1, dans lequel le procédé comprend en outre : la préparation du composé de Formule (VI) par le procédé suivant :
a) la condensation du composé de Formule (VII) avec le composé 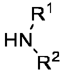 ou son chlorhydrate pour obtenir un composé de Formule (VI) ; 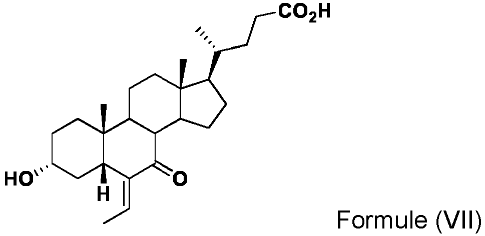 ou
b) en présence d'un acide de Lewis, soumettre le composé de Formule (VIII) à une condensation d'Aldol avec de l'acétaldéhyde pour obtenir le composé de Formule (VI) ; 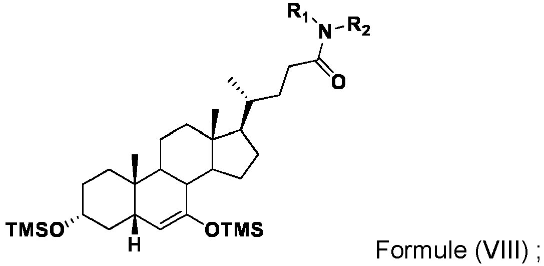
dans lesquelles la définition de chaque groupe est telle que décrite dans la revendication 1.

7. Procédé selon la revendication 6, dans lequel l'agent de condensation à l'étape a) est choisi dans le groupe constitué de N,N'-carbonyldiimidazole (CDI), EDCl, DIC, DCC, HATU, HBTU, TBTU et PyBOP ; de préférence HATU, HBTU et PyBOP ; de préférence PyBOP.

8. Procédé selon la revendication 6, dans lequel le procédé comprend en outre l'étape de : préparation du composé de Formule (VIII) par le procédé suivant :
c) le composé de Formule (IX) est condensé avec le composé 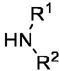 ou son chlorhydrate 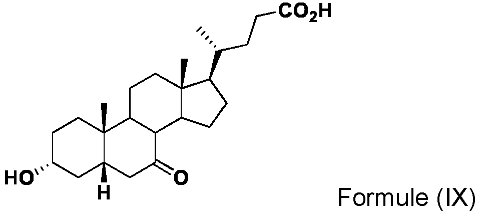 pour obtenir un composé de Formule (X) ; 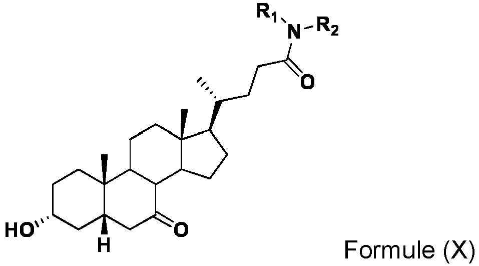
d) en présence d'une base, le traitement du composé de Formule (X) avec du triméthylchlorosilane pour obtenir le composé de Formule (VIII) ;
dans lesquelles la définition de chaque groupe est telle que décrite dans la revendication 1.

9. Procédé selon la revendication 1, dans lequel la formation de sel et la cristallisation comprennent :
(b1) le mélange de l'acide 3α,7α-dihydroxy-6α-éthyl-5β-cholan-24-oïque obtenu à l'étape (a) avec de l'eau pure, l'ajout d'une solution aqueuse d'hydroxyde de sodium, et l'agitation jusqu'à dissolution sensiblement complète pour obtenir une solution transparente ;
(b2) l'ajout lent de la solution aqueuse contenant Mg²⁺ ou Ca²⁺, goutte à goutte, à la solution transparente, et la poursuite de l'agitation jusqu'à ce que la précipitation se produise ;
(b3) la filtration du précipité, le lavage et le séchage sous vide pour obtenir le composé de Formule (A).

10. Procédé selon la revendication 1, dans lequel la formation de sel et la cristallisation comprennent :
(b4) la dissolution de l'acide 3α,7α-dihydroxy-6α-éthyl-5β-cholan-24-oïque obtenu à l'étape (a) dans du méthanol, le goutte à goutte lent d'hydroxyde de sodium dans la solution de méthanol, et l'agitation jusqu'à dissolution sensiblement complète pour obtenir une solution transparente ;
(b5) la concentration de la solution transparente jusqu'à siccité, puis l'ajout d'acétone pour amener à siccité afin d'obtenir un résidu solide ;
(b6) l'ajout d'acétone au résidu et la trituration, la filtration et le séchage sous vide pour obtenir le composé de Formule (A).
